(19) Europäisches Patentamt
European Patent Office
Office européen des brevets

(11) Publication number: **0 131 301**
**A2**

# (12) EUROPEAN PATENT APPLICATION

(21) Application number: 84108010.4

(22) Date of filing: 09.07.84

(51) Int. Cl.⁴: C 07 D 457/02
A 61 K 31/48

(30) Priority: 12.07.83 GB 8318790

(43) Date of publication of application:
16.01.85 Bulletin 85/3

(84) Designated Contracting States:
BE CH DE FR GB IT LI NL

(71) Applicant: FARMITALIA CARLO ERBA S.p.A.
Via Carlo Imbonati n.24
I-20159 Milan(IT)

(72) Inventor: Bosisio, Germano
Via de Santis, 23/B
Palazzo Milanese(paderno dugnano)(IT)

(72) Inventor: Mantegani, Sergio
Via C. Pisacane, 57
Milano(IT)

(72) Inventor: Temperilli, Aldemio
Via Anguissola, 21
Milano(IT)

(72) Inventor: Rossi, Alessandro
Via L. Barzini, 7
Milano(IT)

(74) Representative: Hansen, Bernd, Dr.rer.nat. et al,
Hoffmann, Eitle & Partner Patentanwälte Arabellastrasse 4
D-8000 München 81(DE)

## (54) Anorexigenic ergot derivatives.

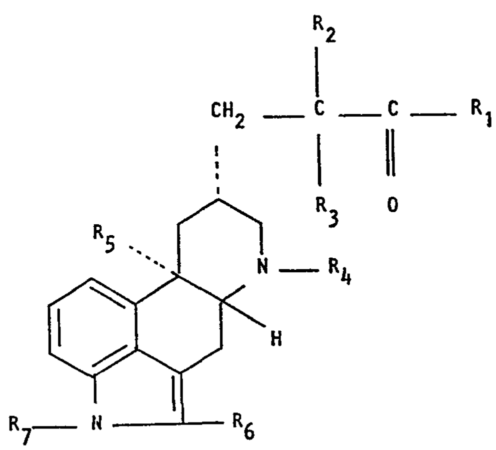

$R_1$ = $CH_3$, phenyl, piperidino, 1-pyrrolidinyl, morpholino, 4-methyl-1-piperazinyl, $C_1$–$C_4$alkyl, $C_1$–$C_4$alkoxy, $NH_2$, NHR' (R'=$C_1$–$C_4$alkyl, cycloalkyl, benzyl or phenyl) or NR''R''' (R'' and R''' independently = $C_1$–$C_4$alkyl),

$R_2$ = H, $C_1$–$C_4$alkyl or phenyl,

$R_3$ = F, Cn, $CHF_2$, $CBrF_2$, $CF_3$, $CH_3S$, $CH_3SO_2$, $NH_2SO_2$, $C_1$–$C_4$alkoxy $C_2$–$C_5$alkanoyl or benzoyl,

$R_4$ = $C_1$–$C_4$ hydrocarbon group,

$R_5$ = H or $CH_3O$,

$R_6$ = H, halogen, $CH_3$ or $CH_3S$, and

$R_7$ = H or $CH_3$.

The 8 α-ergoline derivatives I and their pharmaceutically acceptable salts have anorexigenic properties. Their preparation and compositions containing them are also described.

EP 0 131 301 A2

# Anorexigenic Ergot Derivatives

The invention relates to ergoline derivatives, to processes for their preparation and to pharmaceutical compositions containing them.

The invention provides ergoline derivatives having the general formula I

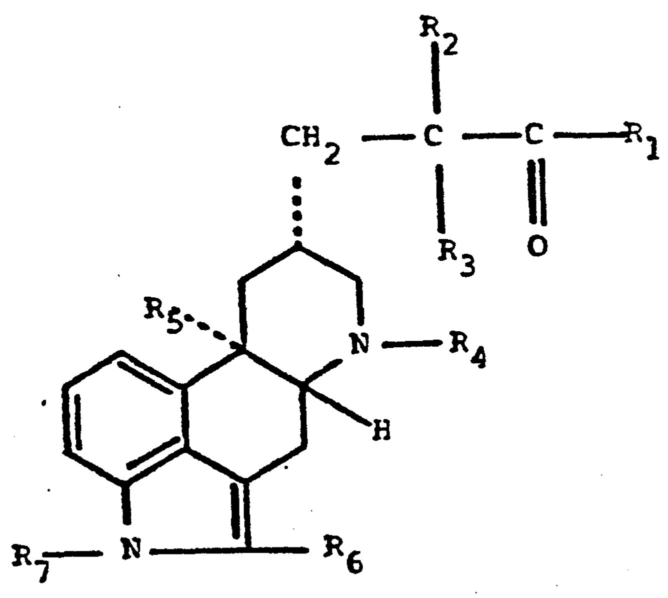

I

wherein $R_1$ represents a methyl, phenyl, piperidino, 1-pyrrolidinyl, morpholino or 4-methyl-1-piperazinyl group, an alkyl or alkoxy group having from 1 to 4 carbon atoms, an amino group, a substituted amino group of the formula NHR' (wherein R' represents an alkyl group having from 1 to 4 carbon atoms, or a cycloalkyl, benzyl or phenyl group) or a substituted amino group of the formula NR" R"' (wherein each of R" and R"'

independently represents an alkyl group having from 1 to 4 carbon atoms);

$R_2$ represents a hydrogen atom, an alkyl group having from 1 to 4 carbon atoms or a phenyl group;

$R_3$ represents a fluorine atom, a cyano, difluoromethyl, difluorobromomethyl, trifluoromethyl, methylthio, methylsulphonyl or sulphonamide group, an alkoxy group having from 1 to 4 carbon atoms, an alkanoyl group having from 2 to 5 carbon atoms or a benzoyl group;

$R_4$ represents a hydrocarbon group having from 1 to 4 carbon atoms;

$R_5$ represents a hydrogen atom or a methoxy group;

$R_6$ represents a hydrogen or halogen atom or a methyl or thiomethyl group;

and

$R_7$ represents a hydrogen atom or methyl group.

The hydrocarbon group represented by $R_4$ may be an alkyl or cycloalkyl group or an unsaturated (ethylenically and/or acetylenically) group. Representative moieties include methyl, ethyl, n-propyl, isopropyl, butyl, t-butyl, isobutyl, cyclopropyl, methylcyclopropyl, vinyl, allyl and propargyl.

The invention further provides a process for the preparation of ergoline derivatives having the general formula I as above defined, the process comprising

condensing an ergoline derivative having the general formula II

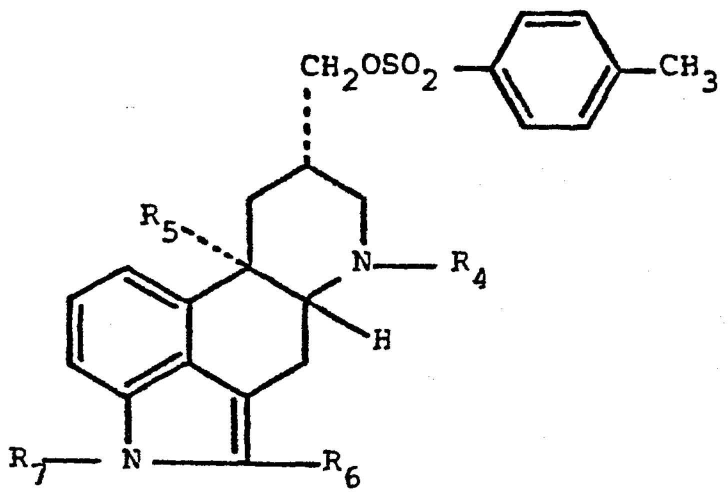

II

wherein $R_4$, $R_5$, $R_6$ and $R_7$ are as above defined with an alkaline salt of a compound having the general formula III

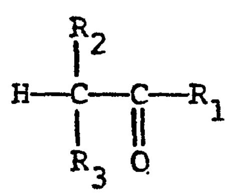

III

wherein $R_1$, $R_2$ and $R_3$ are as above defined.

The ergoline derivatives II are known compounds or can be prepared from known compounds by well known reactions.

The condensation is preferably carried out in a polar aprotic solvent at a temperature of from 50° to 100°C for 2 to 10 hours. Suitable polar aprotic solvents include dimethylsulphoxide and dimethylformamide. The condensation is preferably carried out in the

presence of sodium or potassium iodide. The condensation products may be purified by conventional procedures, chromatography over silica gel being especially suitable.

Numerous semi-synthetic ergot derivatives of this class with $8_{\beta}$-configuration have recently been prepared and shown to possess useful anti-hypertensive activity (see granted European patent 8802). Now we have rather unexpectedly found that the products of 8α-configuration which are the object of the present invention, are useful as anorexigenic agents.

The invention further provides a pharmaceutical composition comprising an ergoline derivative having the general formula I as above defined or a pharmaceutically acceptable salt thereof in admixture with a pharmaceutically acceptable diluent or carrier.

EXAMPLE 1

6-Methyl-8α-(2-ethoxycarbonyl-2-cyano-ethyl)-ergoline

(I: $R_1=OCH_2CH_3$, $R_3=CN$, $R_4=CH_3$ , $R_2=R_5=R_6=R_7=H$).

A mixture of 3.12 g of sodium ethyl cyanoacetate, 8.2 g of of 6-methyl-8α-tosyloxymethyl-ergoline and 3.12 g of potassium iodide in 45 ml of dimethylsulphoxide and 30 ml of ethyl cyanoacetate was heated under stirring

at 90°C for 5 hours. The solution was poured into 2 litres of iced water, and the resultant precipitate was filtered off, dried, and chromatographed on a silica gel column, using chloroform as eluent, to give 4.5 g of the title compound, m.p. 147-148°C.

EXAMPLE 2

6-Methyl-8α-(2-ethoxycarbonyl-3-oxo-butyl)-ergoline

(I: $R_1$=$OCH_2CH_3$, $R_3$=$CH_3CO$, $R_4$=$CH_3$, $R_2$=$R_5$=$R_6$=$R_7$=H).

Operating as in Example 1, but employing sodium ethyl acetoacetate in place of sodium ethyl cyanoacetate, the title compound, m.p. 167-169°C was obtained in 31% yield.

EXAMPLE 3

6-Methyl-8α-(2-ethylcarbamoyl-2-cyano-ethyl)-ergoline

(I: $R_1$=$CH_3CH_2NH$, $R_3$=CN, $R_4$=$CH_3$, $R_2$=$R_5$=$R_6$=$R_7$=H).

A mixture of 0.65 g of sodium N-ethyl-cyanoacetamide, 2 g of 6-methyl-8α-tosyloxymethyl-ergoline, 0.6 g of sodium iodide in 10 ml of dimethylsulphoxide and 2 g of N-ethyl-cyanoacetamide was heated under stirring at 80°C for 10 hours. The solution was poured into 500 ml of water and the resultant precipitate was filtered off, dried, and chromatographed over silica gel to give 0.85 g of the title compound, m.p. 232-233°C.

EXAMPLE 4

6-Methyl-8α-(2-carbamoyl-2-cyano-ethyl)-ergoline

(I: $R_1$=$NH_2$, $R_3$=CN, $R_4$=$CH_3$, $R_2$=$R_5$=$R_6$=$R_7$=H).

Operating as in Example 3, but employing sodium cyanoacetamide in place of sodium N-ethyl-cyanoacetamide, the title compound, m.p. 280-281°C, was obtained in 40% yield.

CLAIMS:

1. An ergoline derivative having the general formula I

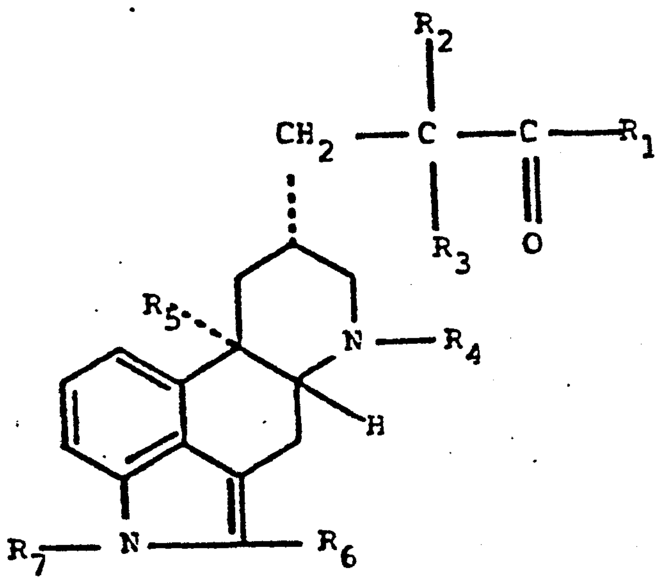

I

wherein $R_1$ represents a methyl, phenyl, piperidino, 1-pyrrolidinyl, morpholino or 4-methyl-1-piperazinyl group, an alkyl or alkoxy group having from 1 to 4 carbon atoms, an amino group, a substituted amino group of the formula NHR' (wherein R' represents an alkyl group having from 1 to 4 carbon atoms, or a cycloalkyl, benzyl or phenyl group) or a substituted amino group of the formula NR" R"' (wherein each of R" and R"'

independently represents an alkyl group having from 1 to 4 carbon atoms);

$R_2$ represents a hydrogen atom, an alkyl group having from 1 to 4 carbon atoms or a phenyl group;

$R_3$ represents a fluorine atom, a cyano, difluoromethyl, difluorobromomethyl, trifluoromethyl, methylthio, methylsulphonyl or sulphonamide group, an alkoxy group having from 1 to 4 carbon atoms, an alkanoyl group having from 2 to 5 carbon atoms or a benzoyl group;

$R_4$ represents a hydrocarbon group having from 1 to 4 carbon atoms;

$R_5$ represents a hydrogen atom or a methoxy group;

$R_6$ represents a hydrogen or halogen atom or a methyl or thiomethyl group;

and

$R_7$ represents a hydrogen atom or methyl group, or a pharmaceutically acceptab'e salt thereof.

2. 6-Methyl-8α-(2-ethoxycarbonyl-2-cyano-ethyl)-ergoline.

3. 6-Methyl-8α-(2-ethoxycarbonyl-3-oxo-butyl)-ergoline.

4. 6-Methyl-8α-(2-ethylcarbamoyl-2-cyano-ethyl)-ergoline.

5. 6-Methyl-8α-(2-carbamoyl-2-cyano-ethyl)-ergoline.

6. A process for the preparation of an ergoline derivative having the general formula I

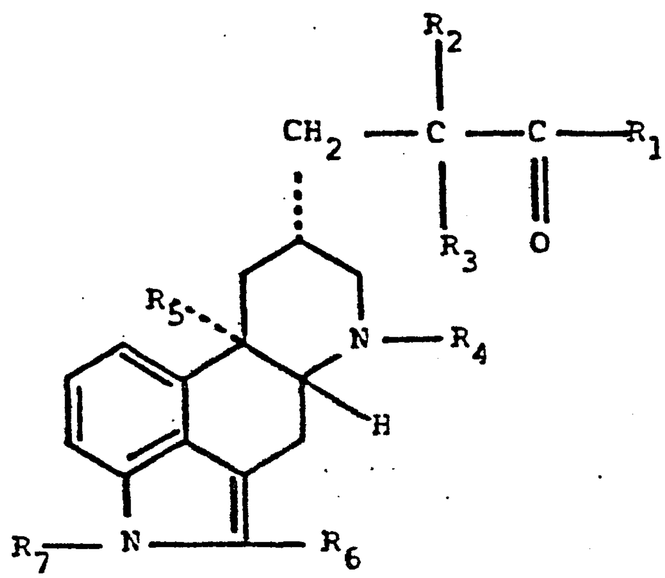

I

as herein defined, the process comprising condensing an ergoline derivative having the general formula II

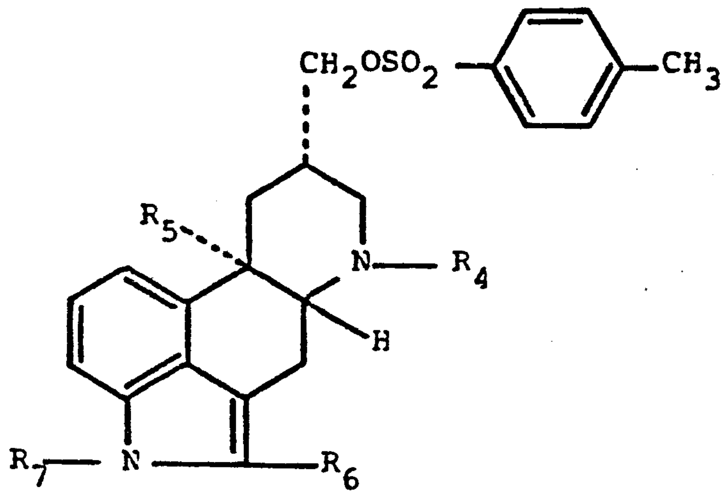

II

as herein defined with an alkaline salt of a compound having the general formula III

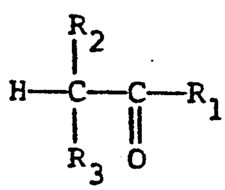

III

as herein defined.

7. A process according to claim 6 in which the condensation is carried out in a polar aprotic solvent.

8. A process according to claim 7 in which the polar aprotic solvent is dimethylsulphoxide or dimethylformamide.

9. A process according to claim 7 or claim 8 in which the condensation is carried out at from 50 to 100°C for 2 to 10 hours.

10. A process according to any of claims 7 to 9 in which the condensation is carried out in the presence of sodium or potassium iodide.

11. A pharmaceutical composition comprising an ergoline derivative having the general formula I as herein defined or a pharmaceutically acceptable salt thereof in admixture with a pharmaceutically acceptable diluent or carrier.